Europäisches Patentamt

(19) European Patent Office

Office européen des brevets

(11) Publication number : **0 455 386 B1**

(12) **EUROPEAN PATENT SPECIFICATION**

(45) Date of publication of patent specification :
29.12.93 Bulletin 93/52

(21) Application number : 91303505.1

(22) Date of filing : 19.04.91

(51) Int. Cl.$^5$ : **A23G 3/00,** C11B 5/00,
**A23G 1/00, A23L 3/3544,**
**A23J 7/00**

(54) Fat based food products.

(30) Priority : 21.04.90 GB 9009018
19.12.90 GB 9027512

(43) Date of publication of application :
06.11.91 Bulletin 91/45

(45) Publication of the grant of the patent :
29.12.93 Bulletin 93/52

(84) Designated Contracting States :
AT BE CH DE DK ES FR GB GR IT LI LU NL SE

(56) References cited :
EP-A- 0 326 829
PATENT ABSTRACTS OF JAPAN vol. 4, no.
113 (C-21)(595) 13 August 1980, & JP-A-55
69688 (TANABE SEIYAKU K.K.) 26 May 1980,
PATENT ABSTRACTS OF JAPAN vol. 12, no.
28 (C-471)(2875) 27 January 1988, & JP-A-62
178521 (TERUMO CORP.) 05 August 1987,

(73) Proprietor : UNITED BISCUITS (UK) LIMITED
Grant House, Syon Lane
Isleworth Middlesex TW7 5NN (GB)

(72) Inventor : Hammond, Eugene William
"Aisling", Warkton
Kettering, Northampton, NN16 9XG (GB)
Inventor : Hicks, Ruth Marian
"Wood End", Shirwell's Hill, Goring Heath
Reading, Berkshire, RG8 7SP (GB)
Inventor : Elsheshtawy, Sally
21 Bourne Court, Station Approach
South Ruislip, Middlesex HA4 6SW (GB)

(74) Representative : Ackroyd, Robert et al
W.P. THOMPSON & CO. Eastcheap House
Central Approach
Letchworth, Hertfordshire SG6 3DS (GB)

EP 0 455 386 B1

## Description

This invention relates to fat-based food products.

It is often desired in the manufacture of fat-based (which term is to be understood in this specification and its claims to include also oil-based) food products such as chocolate and biscuit fillings to disperse an additive material throughout the fat-based food product. In the past, this has been achieved by the formation of an emulsion or solution of the additive material in the fat-based medium. Such emulsions typically comprise water droplets of about 0.5 μm in diameter. The emulsions however often contain at least some droplets of very much larger diameter (up to several tens of micrometers); some droplets could thus have volumes which are up to $10^4$ or more times larger than the smallest droplets. Thus, even if the number of large droplets is small, their total volume is a large proportion of the dispersed additive.

Water-based droplets of the sizes mentioned in the preceding paragraph lead to particular problems in fat systems and in cream and filled biscuits, where the fat-based medium is often in contact with relatively dry biscuit material, for example as in a cream sandwich biscuit. In these circumstances, there is an increasing tendency with increasing droplet size for water droplets to migrate from the filling into the biscuit material, to the detriment of the eating quality of both the biscuit and the filling, or to migrate and coalesce causing phase separation.

In the summary published in Patent Abstracts of Japan, vol.12, no. 28 (C-471) (2875) 27 January 1988, a liposome containing haemoglobin is disclosed. The haemoglobin-containing liposome is useful as an artificial erythrocyte. The aqueous phase of the liposome includes vitamin C in addition to the haemoglobin. The liposome membrane contains vitamin E.

The present invention provides an edible composition comprising an oil- or fat-based edible medium and, dispersed therein, liposomes containing entrained material which comprises vitamin C (ascorbic acid) and, in their lipid structure, vitamin E (tocopherol). The liposomes in such a composition form a stable dispersion which is resistant to migration or separation.

Such a combination is believed to facilitate the known electron-transfer reaction between vitamin C and vitamin E, whereby the vitamin C functions to regenerate vitamin E which is consumed by free radical reactions, the free radicals being formed by oxidative reactions in unsaturated fats and oils. The vitamin E functions as a free-radical acceptor and is oxidised prior to being reduced by vitamin C in the regeneration step, thus increasing its potency.

The liposomes of the composition of the invention may be formed in the presence of vitamins C and E in conditions to introduce vitamin E into the lipid structure of the liposomes and vitamin C to be entrained within the vesicle formed by the lipid structure. Such conditions might typically involve adding vitamin E as neat material to lecithins from which the liposomes are to be formed, mixing by stirring and then forming the liposomes in the presence of an aqueous phase containing vitamin C, in order to entrain vitamin C within the liposomes, and dispersing the liposomes incorporating both vitamins in the oil- or fat- based medium.

In another method, a homogenisation process is used in which lecithins containing vitamin E are blended with an aqueous solution of vitamin C under high shear conditions, for example using an Ultra Turrax or Silverson mixer.

One example of an application of the present invention is in the prevention or retardation of the oxidation of fats and oils, for example edible spreads based on sunflower oil. It is thought that this increased efficiency arises from the presence within the liposomes of an aqueous environment which permits the regeneration of vitamin E to take place even in a medium which has a high fat or oil content or is substantially entirely fat or oil.

The invention thus takes advantage of the known property of lipids such as lecithin to form vesicles known as liposomes which have the ability to occlude or sequestrate relatively large volumes of aqueous materials whilst being themselves compatible with fat based environments.

The diameter of the liposomes of the composition of the invention is advantageously less than 300 nm and preferably less than 100 nm. Diameters as small as 50 nm and even 25 nm are however possible. Larger diameters than 300 nm are also possible in appropriate circumstances. In most cases however the major part of the liposomes will have diameters within the range 50 to 300 nm.

Lecithin (i.e. phosphatidyl choline) preferably forms a major constituent of the lipid component of the liposomes of the composition of the present invention. The liposomes may however be formed in part from other lipids and, indeed, in practice lecithins of the purity grades lower than the highly-purified pharmaceutical grade lecithins (containing 90% or more phosphatidyl choline) are conveniently used. Food grade lecithins having a lecithin content of from about 80% upwards are accordingly suitable. The non-lecithin content of such lecithin compositions is constituted for the most part by other lipids, for example other phospholipids, such as phosphatidyl ethanolamine and lysophosphatidyl choline, and neutral lipids.

It is found that the water-migration problems referred to above can be avoided by use of liposome-based systems, where the very much smaller droplets have a much reduced tendency to migrate into the dry, biscuit material, or to coalesce and separate.

The source of the lipids used in the formation of the liposomes of the compositions according to the present invention can be any suitable source compatible with the edible product applications envisaged. One particularly preferred source is soy bean lecithin which allows a product entirely of vegetable origin to be formed. Other sources include egg lecithin and other lecithins.

The lipids used may be fully hardened lipids, or may be mixtures of hardened and unhardened lipids, some hardened lipid however always being present. Typical mixtures of hardened and unhardened lipids are ones in which the weight ratio of hardened to unhardened lipids is preferably in the range 1:1 to 4:9, the proportion of unhardened lipids preferably being such that the ratio is no greater than 5:8 and more preferably no greater than 6:7.

Suitable commercially-available substantially-pure soy bean lecithin fractions include "Schonat 80" and "Phospholipon 90", both available from Nattermann Phospholipid GmbH (a part of the Rhône-Poulenc Group) and both unhardened lecithins, "Phospholipon 90H" which is available from the same source and is hardened, and "Epicuron 200SH" which is also hardened and is available from Lucas Meyer GmbH of Hamburg. A commercially-available soy bean lecithin containing significant quantities of other lipids but still meeting the criteria for use in a food product is "Bolec FS" which is available from Unimills of Germany. The latter product requires purification before use, for example by acetone precipitation, the resulting product still retaining some of its non-lecithin phospholipid component but being perfectly adequate for food use.

In this specification, the term "hardened lipid" means a lipid which is substantially fully hydrogenated, that is having an iodine value at or close to the minimum possible value of unity. It is of course necessary that, after hardening of unhardened lipids, a purification step be carried out in order to remove any remaining hardening catalyst or other undesirable impurity. Whilst unhardened lipids are often semi-liquid in nature, hardened lipids are usually solids which can, for example, be milled into a fine powder.

The entrained material preferably contains vitamin C in an amount of 10 to 80 mg cm$^{-3}$ of the entrained material, more preferably 20 to 60 mg cm$^{-3}$ and, most preferably about 40 mg cm$^{-3}$. A typical entrained vitamin C phase is therefore vitamin C dissolved in water at a concentration of up to 80 mg cm$^{-3}$.

The lipid structure of the liposomes preferably contains vitamin E in an amount of from 1.5 to 7.1% by weight, more preferably 3.0 to 5.8% by weight and most preferably about 5.1% by weight, of the vitamin E and lipids combined.

The vitamin E used may be in the pure tocopherol form, or in the form of a mixture of tocopherols. The tocopherol must however be in its free form and not in the form of, for example, its acetic or palmitic ester.

The liposomes of the compositions of the present invention can be made by any of the known techniques. This includes hand shaking, homogenisation, ether or alcohol vaporisation techniques, sonication techniques and techniques involving microfluidisation, such as described by H Talsma et al in Drug Development and Industrial Pharmacy 15 (2), 197-207 (1989). The lecithin on which the liposomes is based is preferably pure lecithin but commercially-available lecithin fractions can be used instead. The vitamin C can be incorporated into the liposomes simply by its inclusion at an appropriate concentration in the aqueous phase in which the liposomes are formed by whichever method is chosen. The vitamin E can be incorporated into the lipid structure by addition at a suitable level to the lecithin before combination of the lecithin with the aqueous phase. After preparation, the temperature of the liposomes is maintained sufficiently low, for example below 30°C and, in any event below the transition temperature of the liposomes, preferably at least 5°C therebelow, to maintain their stability.

Formation of the liposome material into a non-aqueous dispersion allows the material, for example in the form of a paste or a dry powder or a fat or oil dispersion (which may be liquid), to be stored and/or transported before its addition to the fat-based product, in which the liposome-based compositions can be dispersed by mixing of the fat-based product and the solid liposome composition, if necessary after warming of the latter. This gives rise to particular advantages compared with the known emulsion techniques where the emulsions must be formed on-site with the food product and are not reliably shelf-stable.

A typical liposome vitamin composition can accordingly be prepared as follows: The following ingredients are placed in a wide-necked vessel, here a 100cm$^3$ beaker:

| | |
|---|---|
| Phosopholipon 90 | 18g |
| Phosopholipon 90H | 8g |
| Vitamin E (tocopherol) | 1.4g |

The above ingredients are combined by stirring with a spatula, before addition of:

| | |
|---|---|
| Aqueous vitamin C composition (vitamin C + water) | 14cm$^3$ |

The ingredients are mixed under high shear conditions using an Ultra Turrax or a Silverson mixer for 5 min ensuring that the mixer probe is brought into contact with all the ingredients so they are thoroughly mixed. The temperature of the mix is maintained throughout at a temperature no higher than 30°C. The resulting product had a highly-whipped texture not unlike a meringue mixture.

To form a biscuit cream suitable for filling sandwich biscuits, the liposome vitamin material is mixed with sugar and fat pre-mixed in the bowl of a Kenwood plenetary mixture and the mixture blended for up to 5 min, again not allowing the temperature to exceed 30°C. The resulting product is a "butter" cream which can be stored or can be used immediately. Sensory testing procedures have shown that products with stable flavours over periods of up to 9 months can be prepared. Tests have also shown that cream sandwich biscuits containing the product can be stored for up to 6 months without any sensorily-detectable softening of the biscuit shells taking place.

EXAMPLES 1 to 10

The invention will now be described further by the following specific examples.

In each example, 1 kg of a typical "butter" cream suitable for use in a sandwich biscuit was prepared by pre-mixing the following ingredients in a Kenwood mixer as described in the general method above.

refined icing sugar        600g

BCF3 cream fat        400g

The cream fat used had a typical solid fat index, as measured on a Brücker "Minispec" pulsed NMR spectrometer as follows:

N20        46-56

N25        10-20

N30        1-6

N32.5        1-3

N35        0-1

To this pre-mix were added respective liposome vitamin compositions prepared as described in the general method above, the compositions varying from example to example as shown in Table 1 which also gives the total weight of liposome vitamin composition added to 0.5 kg of "butter" cream prepared in each case as described above.

In further examples, the Phospholipon 90H can be replaced by Epicuron 200SH and/or the Phospholipon 90 replaced by Schonat 80 or by Bolec FS purified as described above.

Similarly, the butter cream can be replaced by a white confectionery fat and/or the tocopherol replaced a mixture of tocopherols.

Tests carried out using liposome-vitamin compositions as mentioned herein have shown a retarding effect of two-fold to five-fold on the oxidation of the fat-based product.

TABLE 1

| Example | 1 | 2 | 3 | 4 | 5 | 6 | 7 | 8 | 9 | 10 |
|---|---|---|---|---|---|---|---|---|---|---|
| Hardened lecithin[1] | 9g | 8g | 7g | 6g | 5.2g | 5g | 6.5g | 7.5g | 4.5g | 4g |
| Unhardened lecithin[2] | 4g | 5g | 6g | 7g | 7.8g | 8g | 6.5g | 5.5g | 8.5g | 9g |
| Tocopherol addition | 0.7 | 0.7 | 0.7 | 0.7 | 0.7 | 0.7 | 0.7 | 0.7 | 0.7 | 0.7g |
| Vitamin C phase[3] | 20 | 20 | 20 | 20 | 20 | 20 | 20 | 20 | 20 | 20cm$^3$ |
| Amount of liposome antioxidant added to 0.5kg of butter cream | 17g | 17g | 17g | 17g | 17g | 17g | 17g | 17g | 17g | 17g |

1 Phospholipon 90H or Epicuron 200SH
2 Phospholipon 90 or Schonat 80 or Bolec FS (purified as described above)
3 Vitamin C dissolved in water at 40 mg cm$^{-3}$

**Claims**

1.    An edible composition comprising liposomes having an entrained aqueous phase containing an oil- or fat-based edible medium and, disposed herein, vitamin C and, in their lipid structure, vitamin E.

2. A composition according to claim 1, in which lecithin forms a major constituent of the lipid component of the liposomes.

3. A composition according to claim 2, in which lecithin constitutes at least 80% by weight of the lipid component of the liposomes.

4. A composition according to any preceding claim, in which the lipid components of the liposomes comprises hardened and unhardened lipids, the weight ratio of hardened to unhardened lipids being in the range 1:1 to 4:9.

5. A composition according to claim 4, in which the ratio of hardened to unhardened lipids is no greater than 5:8.

6. A composition according to claim 4, in which the ratio of hardened to unhardened lipids is no greater than 6:7.

7. A composition according to any preceding claim, in which substantially all the liposomes have a diameter of less than 300 nm.

8. A composition according to claim 7, in which substantially all the liposomes have a diameter which is also greater than 25 nm.

9. A composition according to any preceding claim, in which vitamin C is present in the entrained aqueous phase of the liposomes at concentration of from 10 to 80 mg cm$^{-3}$.

10. A composition according to claim 9, in which vitamin C is present in the entrained aqueous phase at a concentration of from 20 to 60 mg cm$^{-3}$.

11. A composition according to claim 10, in which vitamin C is present in the entrained aqueous phase at a concentration of about 40 mg cm$^{-3}$.

12. A composition according to any preceding claim, in which vitamin E is present in the lipid structure in an amount of from 1.5 to 7.1% by weight of the vitamin E and lipids combined.

13. A composition according to claim 12, in which vitamin E is present in the lipid structure in an amount of from 3.0 to 5.8% by weight of the vitamin E and lipids combined.

14. A composition according to claim 13, in which vitamin E is present in the lipid structure in an amount of about 5.1% by weight of the vitamin E and lipids combined.

15. A composition according to any preceding claim, in which the edible medium comprises fat and sugar and the composition is suitable for use as a biscuit filling.

16. A biscuit having a filling according to claim 15.

17. A method preventing or retarding oxidation of an oil- or fat-based edible medium, comprising combining lipid material and vitamin E and then forming liposomes in the presence of aqueous phase containing vitamin C, in order to entrain vitamin C within the liposomes, and dispersing the liposomes with the vitamins incorporated therein in the oil- or fat-based medium.

18. A method according to claim 17, in which the edible medium comprises oil or fat and sugar which are combined with each other separately and subsequently combined with the liposomes incorporating the said vitamins.

**Patentansprüche**

1. Eßbare Zusammensetzung umfassend ein eßbares Medium auf Öl- oder Fettbasis und darin dispergiert Liposomen mit einer mitgeführten wäßrigen Phase, die Vitamin C und in deren Lipidstruktur Vitamin E enthält:

2. Zusammensetzung nach Anspruch 1, in der Lecithin einen Hauptbestandteil der Lipidkomponente der Liposomen bildet.

3. Zusammensetzung nach Anspruch 2, in der Lecithin mindestens 80 Gewichtsprozent der Lipidkomponente der Liposomen bildet.

4. Zusammensetzung nach einem der vorausgehenden Ansprüche in der die Lipidkomponenten der Liposomen gehärtete und ungehärtete Lipide umfassen und das Gewichtsverhältnis von gehärteten zu ungehärteten Lipiden im Bereich von 1:1 bis 4:9 liegt.

5. Zusammensetzung nach Anspruch 4, in der das Verhältnis von gehärteten zu ungehärteten Lipiden nicht größer als 5:8 ist.

6. Zusammensetzung nach Anspruch 4, in der das Verhältnis von gehärteten zu ungehärteten Lipiden nicht größer als 6:7 ist.

7. Zusammensetzung nach einem der vorhergehenden Ansprüche, in der im wesentlichen alle Liposomen einen Durchmesser von weniger als 300 nm besitzen.

8. Zusammensetzung nach Anspruch 7, in der im wesentlichen alle Liposomen einen Durchmesser besitzen, der größer als 25 nm ist.

9. Zusammensetzung nach einem der vorhergehenden Ansprüche, in der Vitamin C in der mitgeführten wäßrigen Phase der Liposomen in einer Konzentration von 10-80 mg/cm$^{-3}$ vorliegt.

10. Zusammensetzung nach Anspruch 9, in der Vitamin C in der mitgeführten wäßrigen Phase in einer Konzentration von 20 bis 60 mg/cm$^{-3}$ vorliegt.

11. Zusammensetzung nach Anspruch 10, in der Vitamin C in der mitgeführten wäßrigen Phase in einer Konzentration von etwa 40 mg/cm$^{-3}$ vorliegt.

12. Zusammensetzung nach einem der vorhergehenden Ansprüche, in der Vitamin E in der Lipidstruktur in einer Menge von 1,5 bis 7,1 Gewichtsprozent bezogen auf kombiniertes Vitamin E und Lipide vorliegt.

13. Zusammensetzung nach Anspruch 12, in der Vitamin E in der Lipidstruktur in einer Menge von 3,0 bis 5,0 Gewichtsprozent bezogen auf kombiniertes Vitamin E und Lipide vorliegt.

14. Zusammensetzung nach Anspruch 13, in der Vitamin E in der Lipidstruktur in einer Menge von etwa 5,1 Gewichtsprozent bezogen auf kombiniertes Vitamin E und Lipide vorliegt.

15. Zusammensetzung nach einem der vorhergehenden Ansprüche, in der das eßbare Medium Fett und Zucker umfaßt und die Zusammensetzung für die Verwendung als Biskuitfüllung geeignet ist.

16. Biskuit mit einer Füllung nach Anspruch 15

17. Verfahren zur Verhinderung oder Verlangsamung der Oxidation eines eßbaren Mediums auf Öl- oder Fettbasis, umfassend das Kombinieren von Lipidmaterial und Vitamin E und nachfolgend Ausbildung von Liposomen in Gegenwart einer wäßrigen Phase die Vitamin C enthält, um Vitamin C in den Liposomen mitzuführen und Dispersion der Liposomen mit den darin enthaltenen Vitaminen in dem Medium auf Öl- oder Fettbasis.

18. Verfahren nach Anspruch 17, in dem das eßbare Medium Öl oder Fett und Zucker umfaßt, die getrennt miteinander vereinigt werden und nachfolgend mit den Liposomen, die die Vitamine enthalten, vereinigt werden.

## Revendications

1. Composition comestible comprenant un milieu comestible à base d'huile ou de graisse, et des liposomes, dispersés dans ce milieu et comportant une phase aqueuse entraînée contenant de la vitamine C et, dans leur structure lipidique, de la vitamine E.

2. Composition selon la revendication 1, dans laquelle la lécithine forme un constituant principal du composant lipidique des liposomes.

3. Composition selon la revendication 2, dans laquelle la lécithine constitue au moins 80 % en poids du composant lipidique des liposomes.

4. Composition selon l'une quelconque des revendications précédentes, dans laquelle les composants lipidiques des liposomes comprennent des lipides durcis et non durcis, le rapport pondéral entre des lipides durcis aux lipides non durcis étant situés dans la gamme 1:1 à 4:9.

5. Composition selon la revendication 4, dans laquelle le rapport des lipides durcis aux lipides non durcis n'est pas supérieur à 5:8.

6. Composition selon la revendication 4, dans laquelle le rapport des lipides durcis aux lipides non durcis n'est pas supérieur à 6:7.

7. Composition selon l'une quelconque des revendications précédentes, dans laquelle sensiblement tous les liposomes possèdent un diamètre inférieur à 300 nm.

8. Composition selon la revendication 7, dans laquelle essentiellement tous les liposomes possèdent un diamètre qui est également supérieur à 25 nm.

9. Composition selon l'une quelconque des revendications précédentes, dans laquelle la vitamine C est présente dans la phase aqueuse entraînée des liposomes, en une concentration comprise entre 10 et 80 mg.cm$^{-3}$.

10. Composition selon la revendication 9, dans laquelle la vitamine C est présente dans la phase aqueuse entraînée, en une concentration comprise entre 20 et 60 mg.cm$^{-3}$.

11. Composition selon la revendication 10, dans laquelle la vitamine C est présente dans la phase aqueuse entraînée, en une concentration égale à environ 40 mg.cm$^{-3}$.

12. Composition selon l'une quelconque des revendications précédentes, dans laquelle la vitamine E est présente dans la structure lipidique en une quantité comprise entre 1,5 et 7,1 % en poids de la vitamine E et des lipides, combinés.

13. Composition selon la revendication 12, dans laquelle la vitamine E est présente dans la structure lipidique en une quantité comprise entre 3,0 et 5,8 % en poids de la vitamine E et des lipides, combinés.

14. Composition selon la revendication 13, dans laquelle la vitamine E est présente dans la structure lipidique en une quantité égale à environ 5,1 % en poids de la vitamine E et des lipides, combinés.

15. Composition selon l'une quelconque des revendications précédentes, dans laquelle le milieu comestible comprend de la graisse et du sucre et la composition convient pour être utilisée en tant que matière de fourré pour biscuits.

16. Biscuit possédant un fourré selon la revendication 15.

17. Procédé pour empêcher ou retarder l'oxydation d'un milieu comestible à base d'huile ou de graisse, consistant à combiner une substance lipidique et de la vitamine E, puis à former des liposomes en présence d'une phase aqueuse contenant de la vitamine C, de manière à entraîner la vitamine C à l'intérieur des liposomes, et disperser les liposomes, dans lesquels sont incorporées les vitamines, dans le milieu à base d'huile ou de graisse.

18. Procédé selon la revendication 17, dans lequel le milieu comestible comprend de l'huile ou une graisse et du sucre, qui sont combinés entre eux et sont combinés séparément et ultérieurement aux liposomes contenant lesdites vitamines.